Europäisches Patentamt

European Patent Office

Office européen des brevets

(19)

(11) Publication number: **0 447 256 A2**

(12)

# EUROPEAN PATENT APPLICATION

(21) Application number: 91302240.6

(51) Int. Cl.⁵: **C12M 1/12**

(22) Date of filing: 15.03.91

(30) Priority: 16.03.90 JP 64211/90

(43) Date of publication of application:
18.09.91 Bulletin 91/38

(84) Designated Contracting States:
CH DE FR GB LI SE

(71) Applicant: HITACHI, LTD.
6, Kanda Surugadai 4-chome
Chiyoda-ku, Tokyo 101 (JP)

(72) Inventor: Katoh, Kenji
1611-10 Nishitoyoi
Kudamatsu-shi, Yamaguchi-ken (JP)
Inventor: Nakano, Ryusei
428-1 Hogashistoyoi
Kudamatsu-shi, Yamaguchi-ken (JP)
Inventor: Takai, Masao
1166 Higashitoyoi
Kudamatsu-shi, Yamaguchi-ken (JP)
Inventor: Fujimoto, Masakatsu
6-7-6 Nijigaoka
Hikari-shi, Yamaguchi-ken (JP)

(74) Representative: Stoner, Gerard Patrick et al
Mewburn Ellis 2 Cursitor Street
London EC4A 1BQ (GB)

(54) Methods and apparatus for incubation and sterilization.

(57) Apparatus for continuous incubation of e.g. animal cells, has sterilising filters (25,26,27,28) in the connecting lines (35,36) which transfer liquid incubation medium from a medium supply storage tank (21) to the incubator (22), and from the incubator to a medium extraction tank (23). The sterilising filters prevent the spread of contamination from one element of the apparatus to the next. Sterilisation of the apparatus e.g. by steam (31) need not include pipe portions (B-C ; F-G) between the filters, since contamination in these portions is trapped by the filters. By not sterilising these pipe portions, control units (C1,C2,C3) of the three system elements can function independently of one another in sterilising operations.

EP 0 447 256 A2

## FIG. 2

# METHODS AND APPARATUS FOR INCUBATION AND STERILIZATION

This invention relates to incubation apparatus and methods, typically for incubating biological materials. In particular aspects, it relates to methods and means for achieving and maintaining sterility in an incubation apparatus for a continuous incubation process.

A general incubation process e.g. for animal cells, microorganisms, vegetable cells or the like, comprises keeping the cells or other organisms in a liquid incubation medium, usually under temperature controlled conditions, for a period of time. One important application is in the recovery of substances produced by the organisms when kept in the medium, either for experimental or commercial purposes. An example is the cultivation of cells which have been genetically modified so as to express a useful product e.g. a physiologically active substance.

In a simple batch method, the cells can simply be placed with the liquid medium in a container which has previously been sterilized e.g. by an autoclave, the container closed, and the contents left to incubate for the desired period. At the end of that time, the batch is removed and the relevant product extracted. If more product is needed, the process must be repeated.

For long incubations, or where more product is required, a continuous incubation process is used. In a continuous process, liquid incubation medium in the incubator vessel is at least to some extent exchanged while the incubation proceeds. This exchange can involve supply of fresh medium from a medium supply container, also removal of medium from the incubator unit to remove excretion products of the incubated organisms and also useful products which may have been made by the organisms. With exchange, a higher concentration of organisms in the medium can be maintained so as to maximise growth. The continuous process of this type may go on for days, weeks or even months in certain cases.

Sterility in the incubator vessel is of great importance. Once the incubator becomes contaminated, usually it will be necessary to abandon the incubation and start again, with a great waste of time and of materials.

In a continuous process, particular problems are associated with avoiding contamination when replacing an exhausted medium supply with a fresh one over the course of a long incubation. Although the new medium container and its contents can easily be sterilised, the connection process is liable to lead to contamination and disaster.

In principle, similar problems prevail when removing a full extract container from the discharge of the incubator vessel for replacement with an empty one.

The sterilisation process itself raises further problems. A small apparatus may conventionally be sterilisable as a whole in e.g. a large autoclave, so that the unit containers, the connecting lines between them and indeed also the initial supply of medium in the medium supply container can be sterilised simultaneously. Where the medium itself cannot be heat sterilised, the apparatus can be autoclaved and the medium then charged into the supply container via a sterilising filter. The main difficulties are associated with the re-sterilising or replacement of individual units, as mentioned above. A discussion of apparatus of this type is found in "High Density Incubation of Hybridoma By Filtration-Type Reflux Incubation Tank", Fermentation Engineering Society, Vol 65, No 6, Pages 535-536 (1987).

Where however the whole apparatus is too large for an autoclave the units must be first individually autoclaved. This raises even at the beginning the problems of sterile connection. Alternatively, the system may be sterilised by means of injecting a sterilising fluid (usually steam) into the various vessels and their connecting pipes. A large steam-sterilised system of this type is known. The various connections for medium supply and extraction, and sterilising steam, are extremely complicated and include many tens of control valves. For a number of reasons, each of the medium supply, incubator and extraction units has a respective control sequence for operating its associated valves. Also each unit has its own steam input so that it can be sterilised independently of the others e.g. when recharging the medium supply tank without interrupting the incubation in the incubation tank. Such independent sterilisation causes serious control complications when ensuring that the connecting lines between the units are fully sterilised. For example, sterilisation of the medium supply only, in isolation from the incubator, means that the medium supply control system must operate the upstream valves associated with the incubator system. Otherwise, an intervening portion of the line will not be sterilised. This requires interaction between the control systems, and this necessary interaction causes serious complications in control and also a loss of system flexibility.

In the invention, we aim to provide new methods and apparatus for incubation systems enabling improved control over sterility in the system, and which in particular preferred aspects also offer greater flexibility and simplicity for the sterilising of large-scale apparatus.

In a first aspect, the invention provides incubation apparatus comprising a medium supply unit with a medium supply container for containing a sterilised liquid incubation medium, and connected by a medium supply line into an incubator unit with an incubation vessel, for feeding the medium into the incubator during an incubation process, characterised by means in the medium supply line for sterilising liquid incubation medium passing along it.

Preferably, a medium extraction line from the incubation container to an extract container also comprises sterilising means.

The sterilising means may comprise sterilising filters.

In this way, contamination arising in one of the units of the system cannot spread along the connection line into an adjacent unit. In particular, in the preferred version where the incubator unit comprises sterilising means in both its inlet and its outlet, effective protection is given against contamination of the incubator contents - which may be undergoing a very long incubation - when e.g. the medium supply or medium extraction unit is replenished or exchanged as the case may be. Disconnection points for the lines may therefore be provided in the lines on the side of the filters away from the incubation vessel. If contamination arises in the medium supply for some reason, this cannot reach the incubator unit and therefore the long-term process is not ruined.

In particularly preferred embodiments, sterilising means in the connection line comprise two sterilisers e.g. sterilising filters, on either side of some provision for isolating one unit from the other. In this way, when the two units are so isolated and perhaps physically separated, both units are protected against contamination. When they are connected together again, it is not necessary to sterilise the line portion between the sterilisers since any contamination therein cannot reach either of the units concerned past the sterilisers.

This preferred feature has application particularly in large scale apparatus with provision for individual sterilisation of the units. Each unit can include its own isolation means e.g. a valve in the connection line between the two sterilisers. Each unit can control the closure of its own valve to isolate that unit, but does not need to control the valve associated with the other unit since the portion between the valves need not be sterilised. Any contamination in that portion is unable to pass the sterilisers in either direction. Thus, it is possible for the units to have entirely independent control. This simplifies the system and makes it less expensive. A further, new possibility is the use together in one system of units with incompatible control systems, lending further flexibility.

In a second aspect, the invention provides an incubation method in which liquid incubation medium is passed to and/or from the incubator container of apparatus as described above, through the sterilising means. Particular embodiments of this method involve the disconnection of one unit from an adjacent unit, e.g. for exchange with a replacement, between two sterilisers of such sterilising means.

In a third aspect, the invention provides sterilisation techniques in which one unit of the system is isolated from its adjacent unit or units between two sterilisers in the relevant connection line(s) and sterilised independently of the other unit or units. In preferred versions, the individual unit sterilisation occurs under independent control. For that unit the sterilisation may involve sterilising adjacent units without sterilising a line portion between sterilisers provided in a connection line linking the adjacent units.

In this way, preferred embodiments of the invention make it possible to provide a continuous incubation method and apparatus which can reduce or eliminate one or more of the prior art problems discussed above, in particular the risk of failure of an incubation owing to contamination.

Embodiments of the invention are now described by way of example, with reference to the drawings in which:

Fig 1 shows schematically a bench-scale incubation system, and

Fig 2 shows schematically a large, production-scale incubation system.

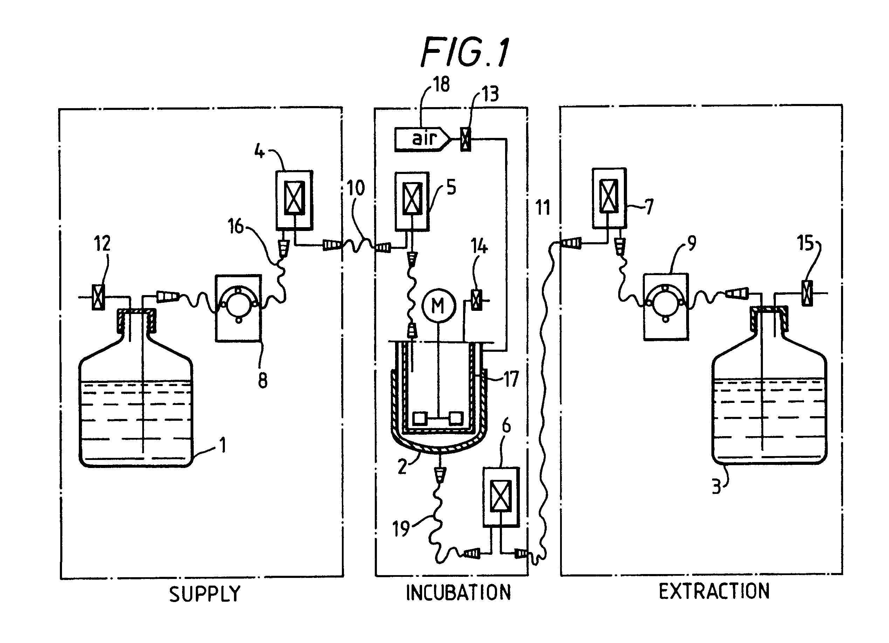

Referring to Fig 1, a simple bench-scale manually-operable continuous incubation apparatus comprises a medium supply unit, an incubation unit, and an extraction unit connected together. The medium supply unit comprises a supply bottle 1 for liquid medium with a sealed top penetrated by an air vent with a sterilising filter 12, and an outlet dip tube of standard type. The dip tube is connected to a flexible transfer tube 16 which also is of standard type e.g. a chemically inert, heat resistant silicone tube. This runs through a roller pump 8 which can pump the liquid medium from the bottle 1 without invading the transfer tube 16. The distal end of the tube 16 is connected sealingly to the inlet of a sterilising filter 4. The sterilising filter is a non-electrically charged membrane-type filter made from polytetrafluorethylene and having a pore size of 0.22 microns. Such a filter is commercially available from Millipore (Registered Trade Mark). These known sterilising filters separate contaminating particles from fluid passing through them, using particle size discrimination. The outlet of the filter 4 is connected via a sealed connection to a short silicone linking tube 10, which in effect is the connection between the supply unit and the incubation unit.

The main component of the incubation unit is an incubation tank 2 for containing organisms to be incubated and their liquid incubation medium under sterile conditions. The tank 2 has a motorised stirrer M, an air intake 18 for air required in the culturing of the organisms, and which receives air through a sterilising filter 13, and an air vent with a further sterilising filter 14.

The incubation unit also comprises sterilisation filters 5,6 in the inlet and outlet connections of the incubation tank 2. The filter 5 on the inlet side of the tank 2 has its intake connected to the first link tube 10 from the supply unit. This filter is identical to the first-described filter 4. Its discharge connects to a further section transfer tube 16 which forms an inlet of the incubation tank 2. The tank 2 contains, in a known manner, a separation membrane 17 for separating cells and liquids in the medium. The discharge at the bottom of the tank 2 is connected by way of another short section of flexible silicone tubing - a first section of an extraction tube designated 19 - to the inlet of a third sterilisation filter 6, the same as the second one. The outlet of this is connected to the extraction unit by way of a second link tube 11 of the same silicone material. Thus, the incubation tank 2 has a sterilising filter connected directly in its medium inlet and in its medium outlet. It is separated by these filters from the nearest stored body of medium both upstream and downstream.

The second link tube 11 connects to the inlet of a fourth sterilising filter 7 whose outlet is connected via a further section of the silicone tube 19 passing through a second roller pump 9 to an inlet dip tube of a bottle 3 for receiving extract medium from the incubator. Like the medium supply container 1, the extract bottle 3 has a vent protected by a sterilising filter 15.

Before operation of the system, the three units are unconnected by the link tubes 10,11. Each unit is individually sterilised in an autoclave. Where the incubation medium is itself heat sterilisable, it may be charged into the bottle 1 before autoclaving. After each unit is taken from the autoclave, any sections of tubing between the containers and their respective sterilising filters are kept sterile by the filters, as are the interiors of the medium supply, incubation and extract containers themselves.

The connecting tubes 10,11 may also be sterilised and connected to the filter connections under sterile conditions e.g. in a flame. This is a known technique. Alternatively, the connection tubes 10,11 need not be sterilised at all. Operation is started by actuating the roller pumps 8,9 so that sterilised medium is supplied from the supply container 1 through the filters 4,5 and via link tube 10 into the incubating container 2. Even when link tube 10 has not been sterilised, any contaminants in that region are unable to escape in either direction because of the filters 4,5. Consequently the medium always arrives in the incubator 2 in a sterile state. In the incubation unit, the culturing medium is subjected to solid-liquid separation by a separation membrane 17, in a known manner following which, by operation of pump 9, it is extracted into the bottle 3 via the two further filters 6,7 in that connection line. Similarly, any contamination arising in the link tube 11 where that has not been sterilised, or has been incorrectly connected, cannot enter either unit past the filters.

In practice, supply and extraction need not necessarily occur either continuously nor simultaneously.

The medium supply system has its sterilisation filter in its medium outlet and in effect constitutes a unit, independently sterilisable, including this filter. The incubation unit comprises, as part of the unit, the sterilisation filter 5 in its medium acceptance line and a second filter 6 in its medium discharge line and again can be sterilised separately. The same applies to the extract bottle 3 with its filter 7.

Where the medium being used cannot be sterilised by heating, it can be charged into the bottle 1 via a sterilisation filter, but this filter is not in the supply line between the bottle 1 and the incubator 2.

If for any reason contamination occurs in the medium supply bottle 1 or in the extract bottle 3, that contamination cannot reach the incubator 2 because of the intervening sterilising filters 4,5,6,7. If the medium supply 1 becomes exhausted and must be replaced by a full, sterile container, the incubator is protected by its inlet filter 5 and the sterility of the entire long-term process is not dependent on the skill used in making the connection with tube 10. Accordingly, the risk of failure of the incubation due to contamination is greatly reduced.

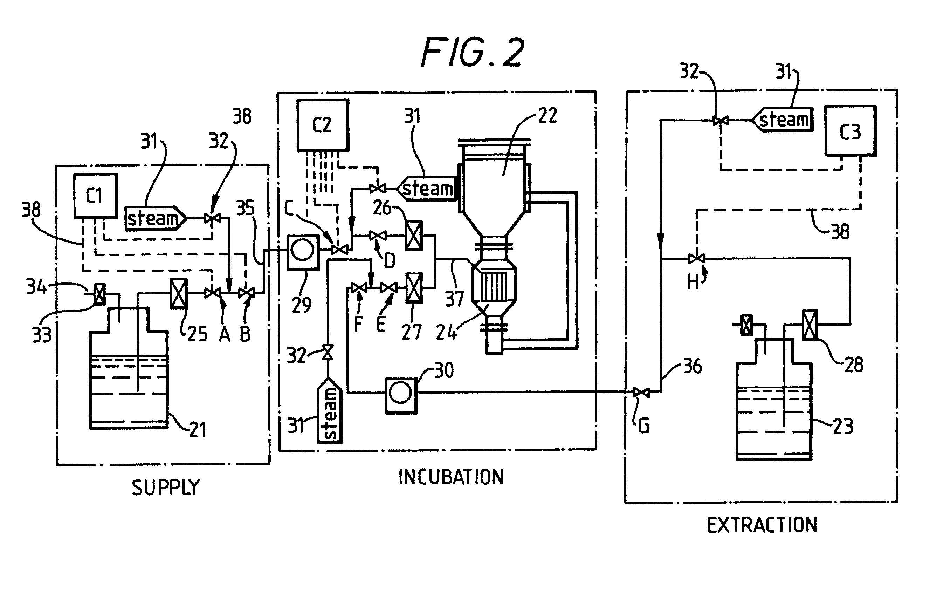

Figure 2 shows schematically a large-scale apparatus for producing incubation products in greater bulk. The essential components correspond to those in the first embodiment described above. That is, a medium supply tank 21 feeds liquid incubation medium to an incubation vessel 22 along connecting pipe 35, while medium is extracted from the incubation tank 22 along extraction pipe 36 into an extract tank 23. In this apparatus, as is commonly the case, the incubation vessel 22 has a divided-off separator chamber 24 for effecting solid-liquid separation.

In the first connecting pipe 35 between supply and incubator, a first sterilising filter 25 is connected immediately downstream of the supply outlet in the supply tank, and a second sterilising filter 26 is connected immediately upstream of the inlet to the incubator vessel chamber 24.

Correspondingly, the second connection pipe 36 to the extractor 23 includes third and fourth sterilising filters 27,28 immediately downstream of the incubator outlet and upstream of the extractor tank intake respectively. A supply pump 29 is provided in the pipe 35 between supply and incubator, and an extract pump 30 between incubator and extract tank 23. These pumps are non-invasive of the system e.g. they may be roller (peristaltic), or they may be a bellows-type pump.

To control flow around the system, it comprises complex valving arrangements. In the schematic diagram, the supply unit is seen to comprise a pair of valves A,B spaced in the connecting pipe 35 downstream of the first filter 25. A supply unit steam source 31 controlled by a steam supply valve 32 has its steam line connected into the connecting pipe 35 between valve A and valve B. Downstream of supply pump 29 in supply pipe 35, but still upstream of the second sterilising filter 26, the incubator unit comprises two further valves C,D in that connecting pipe. An inlet side incubator unit steam source 31, again with its own control valve, is connected into pipe 35 between valve C and valve D.

Supply pipe 35 and extract pipe 36 both meet and communicate into the incubator separator chamber 24 via a common access pipe 37.

In extract pipe 36 and downstream of the third sterilising filter 27 the incubation unit comprises two further control valve stations E,F, with outlet-side incubation unit steam source 31 connected into the pipe 36 between them. Finally, the extraction unit in the extract pipe 36 downstream of extract pump 30 but upstream of fourth sterilising filter 28 comprises two further valves G,H between which the extract unit steam source 31 is connected.

It should be understood that while Fig 2 shows just two valves for each unit in each pipe section, this is schematic and the real case is much more complicated. The supply unit may have twenty to thirty valves, and the incubation unit forty to fifty. These valves include not only flow control valves, but also pressure regulating valves and the like. Because the system is to be fully automated, each unit furthermore comprises pressure sensors, temperature sensors and other sensing and monitoring means known to be needed for automated control of such a system. At least the incubation tank 22 is provided with means (not shown) for maintaining it in a known fashion at desired temperature and pressure.

In each of the three units, the flow control valves A-H, the respective steam sources 31 and other sensors and monitors as mentioned, operate in accordance with control signals from a respective control e.g. a microprocessor control. The supply unit has control C1, the incubation unit control C2 and the extraction unit control C3. These are operatively connected to the various valves of their own units, in a manner which itself is known. They are programmed with the information necessary to open, close and adjust the valves in accordance with a selected medium supply and extraction scheme appropriate to the incubation being carried out. However the controls C1,C2,C3 do not interact with one another. Indeed, they may be incompatible both as to hardware and as to software. Each control sends the necessary sequencing signals only to its own valves and, when appropriate, sterilising steam supply 31 via connections 38 indicated schematically.

Sterilisation of the system is now considered. Each unit or line should be sterilised by its own steam supply. First, it is of interest to consider the sterilisation as in the prior art i.e. in the case that filters 25-28 are absent. To sterilise the supply, control C1 closes valve B and admits steam from the supply steam source 31 by way of control valve 32. This sterilises the entire supply unit upstream of valve B. For the incubation unit, closure of valve C and valve F under control C2 followed by admission of steam from the inlet side and outlet side steam sources 31 sterilises the incubation container 22 and also the piping between the named valves. The extraction unit under control C3 can close its valve G and be sterilised in a manner analogous to the supply unit.

With each system operating independently in this way, however, the piping sections between valve B and valve C, and between valve F and G are not sterilised. In the prior art, the first-mentioned line segment must be sterilised by operating the supply unit steam source and the inlet side incubation unit steam source, while closing valves A and D and opening valves B and C. This however requires operation of the two steam sources, and of the named valves, in synchronism between the two units. That is, sequencing information must pass between controls C1 and C2 and indeed this is how the prior art system must operate. The controls cannot be independent; they must be compatible and interact.

Furthermore, the prior art large-scale system is liable to all the difficulties with contamination, after incubation has started, as described above for the small-scale version.

In the embodiment, however, the sterilising filters 25-28 are included. As in the first embodiment these are non-electrostatically charged membrane-type PTFE filters of pore size 0.22 microns. Other types may be suitable e.g. charged membrane-type, depth-type filters, or (as these become available and suitable) a centrifugal separator or even gel filtration means.

In the same way as described for the first embodiment, these filters prevent the spread of contamination in the system and in particular protect the incubator unit.

Sterilisation in this embodiment of the invention can proceed as follows. The supply unit can close its valve B and sterilise using supply unit steam source 31. Incubator unit closes valve C and valve F and sterilises using its inlet and outlet-side steam sources. The extraction unit can sterilise by closing its valve G and using its own steam source 31. As before, this leaves the pipe sections between valve B and valve C, and between valve F and valve G, unsterilised. To sterilise those pipe sections would require synchronised operation between the control systems of adjacent units. However the control systems are independent, rendering the apparatus much simpler to operate, and cannot synchronise. Because of the feature of the invention, namely the filters disposed in the connection pipes, any contamination in the unsterilised pipe portions referred to cannot adversely affect the incubation process, even when medium exchange is carried out.

In the manner described above, it is possible to sterilise only the supply unit, or only the extraction unit, during an incubation process when the incubator unit cannot be disturbed, with greatly reduced risk of contamination to the incubator while having a more simple sterilisation operation than previously.

Furthermore, it will be understood that the control independence provided by the described system enables the interchange e.g. of one supply unit between two different incubation systems which may be of different type, since there is no need for control interaction between the supply and incubation units in the system described.

## Claims

1. Incubation apparatus comprising an incubation vessel (2,22) for holding incubating matter in a continuous incubation process, and a medium supply container (1,21) for holding sterilised liquid medium for supply to the incubation vessel (2,22) during said process, characterised in that sterilising means (4,5;25,26) are provided in a medium supply line (16,10;35) along which the medium is to be supplied from the supply container (1,21) to the incubation vessel (2,22).

2. Incubation apparatus according to claim 1 in which further sterilising means (6,7;27,28) are provided in a medium extraction line (17,11;36) along which medium is to be extracted from the incubation vessel (2,22) in the incubation process.

3. Incubation apparatus according to claim 2 in which the medium extraction line leads to an extraction container (3,23).

4. Incubation apparatus according to any one of the preceding claims in which said sterilising means comprise a sterilising filter (4-7;25-28).

5. Incubation apparatus according to any one of the preceding claims in which the sterilising means in the medium supply line and/or medium extraction line comprise two sterilisers spaced along said line or lines.

6. Incubation apparatus according to claim 5 comprising means (10,11;A-H) providing for interruption of the line or lines between the sterilisers.

7. Incubation apparatus according to claim 6 in which

the interruption means for the medium supply line between its sterilisers (25,26) comprise a first interrupter (B) towards the medium supply and operable by a medium supply control (C1) but not by an incubation control (C2), and a second interrupter (C) towards the incubation vessel (22) and operable by the incubation control (C2) but not by the medium supply control (C1),

and/or

the means for interrupting the medium extraction line (36) between the sterilisers (27,28) thereof comprise a first interrupter (F) towards the incubation vessel (22) and operable by the incubation control (C2) but not by a medium extraction control (C3), and a second interrupter (G) operable by the medium extraction control (C3) but not by the incubation control (C2).

8. Incubation apparatus according to claim 7 in which each said control unit (C1,C2,C3) is capable of actuating means (31 and/or 32) for injecting sterilising fluid into its respective container (21,23) or vessel (22) as the case may be.

9. A continuous incubation process in which matter is incubated in a liquid incubation medium in the incubation vessel (2,22) of apparatus according to any one of claims 1 to 8, and in which liquid medium is passed from the medium supply container (1,21) of the apparatus into the incubation vessel (2,22) through the sterilising means in the medium supply line (16,10;35).

10. A process according to claim 9 in which the apparatus is in accordance with claim 3 and liquid medium is extracted from the incubation vessel (2,22) through the sterilising means in the medium extraction line (17,11;36) to the extraction container (3,23).

11. A process according to claim 9 or claim 10 in which, before the incubation begins, the medium supply container (1,21), incubation vessel (2,22) and, where present, the extraction container (3,23) are sterilised.

12. A process according to any one of claims 9 to 11 in which the vessels and container are sterilised independently of one another, with the incubation vessel (2,22) isolated from contamination by the sterilising means in the medium supply line (16,10;35) and the medium extraction line (17,11;36) connected thereto.

13. A process according to any one of claims 9 to 12 in which, during the progress of incubation of the matter in the incubation vessel (2,22), the medium supply container (1,21) and/or the medium extraction container (3,23) (where present) is isolated from the incubation vessel (2,22) and replenished with fresh medium, or replaced with another such container (1,21,3,23) with the incubation vessel (2,22) separated from the isolation location by said sterilising means.

14. A method of sterilising a continuous incubation apparatus, which apparatus comprises

a medium supply container (21), an incubation vessel (22), a medium supply line (35) between the medium supply container and incubation vessel, with two sterilisers (25,26) in the medium supply line (35),

and optionally also a medium extraction container (23), a medium extraction line (36) from the incubation vessel (22) to the medium extraction container (23), and two sterilisers (27,28) in the medium extraction line (36),

the method comprising

(a) interrupting the medium supply line (35), and also optionally the medium extraction line (36) when present, between the sterilisers thereof;

(b) sterilising one or more of the medium supply container (21), the incubation vessel (22) and the medium extraction container (23), and

(c) restoring communication along the interrupted line or lines between the sterilising means.

15. A method according to claim 14 in which the sterilisations of respective vessel (22) and/or containers (21,23) are carried out under respective independent controls.

FIG.1

SUPPLY    INCUBATION    EXTRACTION

FIG.2

EXTRACTION

INCUBATION

SUPPLY